# EUROPÄISCHE PATENTSCHRIFT

(11) **EP 0 542 780 B1**
(45) Veröffentlichungstag und Bekanntmachung des Hinweises auf die Patenterteilung: **12.04.1995**
(21) Anmeldenummer: 91913570.7
(22) Anmeldetag: 22.07.1991
(51) Int. Cl.: C07C 17/20, C07C 19/08

(54) **VERFAHREN ZUR HERSTELLUNG VON WEITGEHEND FLUORIERTEN ALKYLBROMIDEN**
PROCESS FOR PRODUCING HIGHLY FLUORINATED ALKYL BROMIDES
PROCEDE POUR LA FABRICATION DE BROMURES D'ALKYLE LARGEMENT FLUORES

(30) Priorität: 09.08.1990 DE 4025227
(43) Veröffentlichungstag der Anmeldung: 26.05.1993
(73) Patentinhaber: RIEDEL-DE HAEN AKTIENGESELLSCHAFT, D-30926 Seelze (DE)
(72) Erfinder: WINTERFELDT, Andreas, D-3013 Barsinghausen 1 (DE); BARTELS, Günter, D-3006 Gro burgwedel (DE); KNIEPS, Reinhard, D-3000 Hannover 91 (DE)
(74) Vertreter: Muley, Ralf, Dr.
(86) Internationale Anmeldenummer: EP9101376
(87) Internationale Veröffentlichungsnummer: WO9202475

(56) Entgegenhaltungen:
- EP-A- 0 298 870
- CH-A- 605 492
- HOUBEN-WEYL: "Methoden der organischen Chemie", edition 4, vol. V/4: "Halogenverbindungen", 1960, pages 354-360, Georg Thieme publischer Stuttgart, DE

## Beschreibung

Die vorliegende Erfindung betrifft ein Verfahren zur Herstellung von weitgehend fluorierten Alkylbromiden, insbesondere von Perfluoralkylbromiden, ausgehend von weitgehend fluorierten Alkyliodiden, insbesondere von Perfluoralkyliodiden.

Weitgehend fluorierte Alkylbromide, insbesondere Perfluoralkylbromide werden, z.B. als Zwischenprodukte für die Herstellung von polymeren Flüssigkeiten, Harzen und Elastomeren, als Röntgenkontrastmittel, zur Herstellung von pharmazeutischen Präparaten und in wäßriger Emulsion als Blutersatzstoff verwendet. Ein als Blutersatzstoff bevorzugtes Perfluoralkylbromid ist das Perfluoroctylbromid.

Für die Herstellung von Perfluoralkylbromiden sind bereits eine Reihe von Verfahren bekannt. So werden z.B. gemäß der japanischen Patentschrift JP 60 184 033 (C.A. Vol 104 (1986), 88106p) Perfluoralkyliodide in Anwesenheit von Radikalinitiatoren mit elementarem Brom zu Perfluoralkylbromiden umgesetzt. Hazeldine (J. Chem. Soc. 1953, 3761 - 3768) beschreibt auf der Seite 3763 und 3766 die Umsetzung von Perfluoralkyliodiden mit elementarem Brom unter Bestrahlung mit UV-Licht. Beide Verfahrensweisen sind durch den Einsatz von elementarem Brom, durch das Freisetzen von elementaren Iod, Interhalogenverbindungen sowie Fluorwasserstoff mit erheblichen Werkstoff- und sicherheitstechnischen Problemen verbunden.

Weitere Herstellungsverfahren für Perfluoralkylbromide sind z.B. (R_{F}= Perfluoralkyl): Die Einwirkung von Brom auf Verbindungen R_{F} - SF₅ bei 500°C in Gegenwart von Nickel (USP-3456024); die Einwirkung von Brom auf Verbindungen R_{F} -SO₂Na in Gegenwart von KI/I₂ (C.A., Vol. 107 (1987), 236043); die Einwirkung von Brom auf Salze von perfluorierten Carbonsäuren (USP-2678953), insbesondere auf R_{F}COOAg (USP-2678953 und Hauptschein et al., J. Am. Chem. Soc. 74 (1952), 1347ff); die Einwirkung von Brom auf Verbindungen R_{F}H bei gleichzeitiger Bestrahlung mit UV-Licht (J. Chem. Soc. 1953, 3761). Bei all diesen Verfahren treten durch die Verwendung von elementarem Brom erhebliche Werkstoff-und sicherheitstechnische Probleme auf. Darüberhinaus sind die Ausgangsverbindungen schwer zugänglich oder müssen über eine zusätzliche Verfahrensstufe aus den entsprechenden Perfluoralkyliodiden hergestellt werden. Dies gilt auch für die Herstellung von Perfluoralkylbromiden durch Umsetzung von R_{F}SO₃Cl mit HBr-Gas in Gegenwart eines Katalysators bei 125°C. J. Am. Chem. Soc. (EP-A1-0298870).

Nach Fainberg et al. 79. 4172 (1957) kann Perfluoralkylbromid durch Umsetzung von Perfluoralkyliodid mit Lithiumbromid in Aceton hergestellt werden. Die naheliegende Übertragung dieser Umhalogenierung auf andere Perfluoralkyliodide gelingt aber nicht, weil in normalen Perfluoralkyliodiden keine Aktivierung des Iods durch eine Allylgruppe vorliegt. Wie aus dem nachfolgenden Vergleichsbeispiel 1 hervorgeht, sind die von Fainberg et al. beschriebenen Reaktionsbedingungen auf Perfluoroctyljodid nicht erfolgreich übertragbar. Die nachfolgenden Vergleichsbeispiele 2 und 3 zeigen, daß auch der Versuch einer Reaktionsbeschleunigung unter Phasentransfer-Katalyse zu keinem befriedigenden Ergebnis führt.

Die Vergleichsbeispiele entsprechen in ihrem Ergebnis den Erwartungen, da bekannt ist, daß Fluoratome die Reaktivität von Alkylhalogeniden in nucleophilen Austausch-Reaktionen deutlich herabsetzen, (vgl. z.B. Houben-Weyl, Methoden der organischen Chemie, 4. Aufl., Bd. 5/4, S. 685, 688).

Die bisher bekannten Verfahren zur Herstellung weitgehend fluorierter Alkylbromide, insbesondere von Perfluoralkylbromiden sind aus den vorstehend genannten Gründen nicht befriedigend. Es ist daher Aufgabe der vorliegenden Erfindung, ein technisch einfaches Verfahren zur Herstellung von weitgehend fluorierten Alkylbromiden, insbesondere von Perfluoralkylbromiden, ausgehend von den leicht zugänglichen weitgehend fluorierten Alkyliodiden, insbesondere von Perfluoralkyliodiden, zur Verfügung zu stellen.

Überraschenderweise wurde gefunden, daß bei Anwendung äquimolarer Mengen eines in der Bromidform vorliegenden Phasentransferkatalysators weitgehend fluorierte Alkylbromide, vorzugsweise Perfluoralkylbromide in einer einfachen Reaktion aus den entsprechenden weitgehend fluorierten Alkyliodiden, vorzugsweise Perfluoralkyliodiden, zugänglich sind.

Die Erfindung betrifft ein Verfahren zur Herstellung von fluorierten Alkylbromiden der Formel I

X-CₙF₂ₙ-Br (I)

worin X = H, F oder (F₃C)₂CF- und n = 1 bis 20 bedeuten, ausgehend von fluorierten Alkyliodiden der Formel III

X-CₙF₂ₙ-I (III)

worin X und n wie oben definiert sind, dadurch gekennzeichnet, daß ein fluoriertes Alkyliodid der Formel III mit einem in der Bromidform vorliegenden Phasentransferkatalysator im Molverhältnis 1 : (0,4 bis 3) umgesetzt wird.

In den Formeln I und III bedeutet n vorzugsweise 4 bis 16 und besonders bevorzugt 6 bis 12.

In den Formeln I und III besitzt die Gruppe -CₙF₂ₙ-insbesondere die Form

-(CF₂)ₙ- (II).

In den Formeln I und III bedeutet X vorzugsweise (F₃C)₂-CF- und besonders bevorzugt F. Das erfindungsgemäße Verfahren ist daher besonders zur Herstellung von Perfluoralkylbromiden, besonders bevorzugt solcher mit 6 bis 12 C Atomen, ganz besonders bevorzugt zur Herstellung von Perfluoroctylbromid, geeignet.

Die als Ausgangsverbindungen benutzten Verbindungen der Formel III, sind bekannt oder lassen sich nach verschiedenen für diese Verbindungsklasse bekannten Verfahren herstellen.

Als Phasentransferkatalysator können alle organischen Verbindungen eingesetzt werden, in denen Brom in anionischer Form vorliegt und die eine ausreichende Lös lichkeit im Reaktionsmedium besitzen. Geeignete Phasentransferkatalysatoren sind insbesondere Bromide quaternärer organischer Verbindungen, wie z.B. quaternärer Ammonium-, Phosphonium- und Arsoniumverbindungen der allgemeinen Formeln IV, V und VI:

R₄N^{⊕}Br^{⊖} (IV) R₄P^{⊕}Br^{⊖} (V) R₄As^{⊕}Br^{⊖} (VI)

In derartigen Phasentransferkatalysatoren können die vier Reste R gleich oder verschieden sein und/oder auch funktionale Gruppen aufweisen. Die Reste R können z.B. Alkylreste mit 1 bis 20 C-Atomen, Phenyl- oder Benzylreste sein. Als zentrales Onium-Atom können außer Stickstoff, Phosphor und Arsen auch andere Atome, wie z.B. Antimon oder Schwefel in Betracht kommen. Geeignete Phasentransferkatalysatoren sind z.B.:
(CH₃)₄NBr, (C₂H₅)₄NBr; (C₃H₇)₄NBr; (C₄H₉)₄NBr; (C₈H₁₇)₃NCH₃Br; C₆H₅CH₂N(C₂H₅)₂Br; C₆H₁₃N(C₂H₅)₃Br; C₈H₁₇N(C₂H₅)₃Br; C₁₀H₂₁N(C₂H₅)₃Br; C₁₂H₂₅N(C₂H₅)₃Br; C₁₆H₃₃N(CH₃)₃Br; C₁₆H₃₃N(C₂H₅)₃Br; (C₆H₅)₄PBr; (C₆H₅)₃PCH₃Br; (C₈H₁₇)₃PC₂H₅Br; C₁₆H₃₃P(C₂H₅)₃Br.

Bevorzugt werden quaternäre Ammonium- oder Phosponiumbromide eingesetzt. Besonders bevorzugt sind quaternäre Phosphoniumbromide, insbesondere Tetraalkylphosphoniumbromide, mit 1 bis 20 C-Atomen in den einzelnen Alkylresten, wie z.B. Tetrabutylphosphoniumbromid. Es kann auch ein Gemisch verschiedener Phasentransferkatalysatoren eingesetzt werden.

Die Herstellung der genannten Phasentransferkatalysatoren ist bekannt. Viele sind handelsüblich.

Die erfindungsgemäße Umsetzung wird durch einfaches Vermischen des fluorierten Alkyliodids der Formel III mit dem Phasentransferkatalysator durchgeführt. Der Zusatz eines Lösungsmittels ist nicht erforderlich. Die Umsetzung kann jedoch auch in einem geeigneten Lösungsmittel oder Lösungsmittelgemisch durchgeführt werden.

Pro Mol fluoriertem Alkyliodid der Formel III werden vorzugsweise 0,5 bis 2 mol und ganz besonders bevorzugt 0,8 bis 1,4 mol Phasentransferkatalysator in der Bromidform eingesetzt. In vielen Fällen beträgt das Molverhältnis fluoriertes Alkyliodid der Formel III: Phasentransferkatalysator = 1 : 1 oder etwa 1 : 1.

Der Phasentransferkatalysator kann als Feststoff oder auch in Form einer wäßrigen Lösung eingesetzt werden. Wird er als wäßrige Lösung eingesetzt, dann wird das Wasser vor der eigentlichen Umsetzung aus dem Reaktionsgemisch abdestilliert. Diese Abdestillation des Wassers erfolgt zweckmäßigerweise azeotrop unter Rückführung der organischen Phase in das Reaktionsgemisch.

Die Reaktionstemperatur liegt zwischen 0°C und dem Siedepunkt des Reaktionsgemisches unter Normaldruck. Die Reaktion wird insbesondere bei einer Temperatur von 20°C bis zum Siedepunkt des Reaktionsgemisches unter Normaldruck, vorzugsweise bis zum Siedepunkt des weitgehend fluorierten Alkyliodids der Formel III unter Normaldruck, durchgeführt. In vielen Fällen wird die Umsetzung bei Temperaturen von 50 bis 140°C durchgeführt. Die Umsetzungsgeschwindigkeit ist, wie üblich, bei höheren Temperaturen größer als bei niederen.

Es kann zweckmäßig sein, die Reaktion unter Inertgasatmosphäre, z.B. unter Argon, durchzuführen.

Die Umsetzung bzw. Aufarbeitung kann auf verschiedene Weise erfolgen und z.B. so durchgeführt werden, daß gebildetes fluoriertes Alkylbromid der Formel I während der Reaktion oder nach der Reaktion abdestilliert wird. Man kann auch vorzugsweise so aufarbeiten, daß nach der Umsetzung die Temperatur des Reaktionsgemisches gegebenenfalls erniedrigt und das Reaktionsgemisch mit Wasser versetzt, d.h. durchmischt wird, und danach das Gemisch in eine wäßrige und eine organische Phase getrennt wird und die abgetrennte organische Phase destillativ in ihre Bestandteile, d.h. insbesondere in fluoriertes Alkylbromid der Formel I und nicht umgesetztes fluoriertes Alkyliodid der Formel III zerlegt wird. Das bei dieser Aufarbeitung zurückgewonnene Ausgansprodukt kann der Reaktion von neuem zugeführt werden.

Der nach Beendigung der Reaktion als Iodid/Bromid-Gemisch vorliegende Phasentransferkatalysator läßt sich nach üblichen Methoden wieder in das reine Bromid zurückführen. Bei der bevorzugten Aufarbeitung des Reaktionsgemisches (Versetzung des Reaktionsgemisches mit Wasser, Phasentrennung) enthält die zurückbleibende wäßrige Phase den Phasentransferkatalysator als Iodid/Bromid-Gemisch.

Zur Überführung des Iodanteils in die Bromidform, kann die wäßrige Phase einem Ionenaustausch, bei dem Iodid gegen Bromid ausgetauscht wird, unterzogen werden. Hierfür geeignete polymere Ionenaustauscher sind bekannt. Die bei dem Ionenaustausch anfallende wäßrige Lösung des Phasentransferkatalysators in der Bromidform kann, wie bereits erwähnt, direkt für die erfindungsgemäße Umsetzung eingesetzt werden. Es ist aber auch möglich, aus der wäßrigen Lösung den Phasentransferkatalysator in fester Form zu isolieren und ihn dann von neuem für die Durchführung der erfindungsgemäßen Reaktion einzusetzen.

Bei dem erfindungsgemäßen Verfahren werden die gewünschten fluorierten Alkylbromide der Formel I, vorzugsweise die Perfluoralkylbromide, in Ausbeuten bis über 90 % (bezogen auf umgesetzes Ausgangsprodukt) erhalten. Das nicht umgesetzte Ausgansprodukt kann auf einache Weise zurückgewonnen und von neuem eingesetzt werden. Der Phasentransferkatalysator in der Bromidform wird bei der erfindungsgemäßen Umsetzung nicht als Katalysator, sondern als Reaktionskomponente eingesetzt und bei der Reaktion zum großen Teil in die entsprechende Iodidform umgewandelt. Die Iodidform kann wieder leicht in die Bromidform zurückgewandelt und dann von neuem eingesetzt werden. Die gaschromatographisch bestimmten Reinheiten der hergestellten Endprodukte sind hoch und liegen in vielen Fällen über 99 %.

An Hand der nachstehenden Beispiele wird die Erfindung weiter erläutert:

### Beispiel 1

986 g Perfluoroctyliodid (1,81 mol) und 613 g Tetrabutylphosphoniumbromid (1,81 mol) werden 8 Stunden am Rückfluß gekocht.

Bei einer Sumpftemperatur von maximal 160°C wird darin langsam bei einer Kopftemperatur von 140-143°C Perfluoroctylbromid abdestilliert. Anschließend wird im Vakuum (18 mbar) weiter aus dem Reaktionsgemisch abdestilliert.

Dem Sumpf werden nach Abkühlen auf 90°C 250 ml Wasser zugesetzt und nicht umgesetztes Perfluoroctyliodid azeotrop abdestilliert. Nach der Phasentrennung werden die Destillate vereinigt, mit Natriumdisulfit gewaschen und anschließend fraktioniert destilliert.
- Ausbeute:: 540 g Perfluoroctylbromid,
340 g Perfluoroctyliodid, das sind
60 % d. Th. Perfluoroctylbromid oder
91 % d. Th bezogen auf umgesetztes Perfluoroctyliodid
- Kp:: 142 - 143°C
- GC:: Reinheit größer 99 %
Der verbleibende Rückstand von 500 g wird in insgesamt 4500 g 20%igem Methanol gelöst, mit 5 g Aktiv-Kohle ausgerührt, filtriert und ist so für eine Regeneration an polymeren Ionenaustauschharzen geeignet.

### Beispiel 2

Bei einer Wiederholung des Beispiels 1 wird das Tetrabutylphosphoniumbromid in Form einer wäßrigen Lösung eingesetzt. Vor der eigentlichen Reaktion wird das Wasser unter Rückführung der organischen Phase azeotrop abdestilliert. Dann wird das Reaktionsgemisch 18 Stunden lang auf 140°C erhitzt, dann auf Raumtemperatur abgekühlt und mit 500 ml Wasser vermischt. Die Phasen werden getrennt und die organische Phase mit Natriumdisulfit gewaschen und anschließend fraktioniert destilliert.
- Ausbeute:: 530 g Perfluoroctylbromid
345 g Perfluoroctyljodid, das sind
58 % d. Th. Perfluoroctylbromid oder
90 % d. Th. bezogen auf umgesetztes Perfluoroctyljodid
Kp: 142 - 143 °C
GC: Reinheit größer 99 %
Das nachfolgende Vergleichsbeispiel zeigt, daß die von Fainberg et al J. Am. Chem. Soc. 79, 4172 (1957) für die Herstellung von Perfluorallylbromid angegebenen Reaktionsbedingungen sich nicht auf die Herstellung von Perfluoroctylbromid übertragen lassen. Die nachfolgenden Vergleichsbeispiele 2 und 3 zeigen, daß auch die Durchführung der Reaktion unter Phasentransferbedingungen keine brauchbaren Ausbeuten an Perfluoroctylbromid ergibt.

### Vergleichsbeispiel 1

Eine Lösung von 19 g LiBr (220 mmol) in 150 ml trockenem Aceton wird innerhalb von 10 Minuten mit 100 g Perfluoroctyliodid (183 mmol) versetzt und danach 8 Stunden am Rückfluß gekocht. Man gießt in 500 ml Wasser, trennt die organische Phase ab und trocknet mit wenig CaCl₂.
- Ausbeute:: 100 g Perfluoroctyljodid 95%ig.

Perfluoroctylbromid ist gaschromatographisch nicht nachweisbar.

### Vergleichsbeispiel 2

100 g Perfluoroctyliodid (183 mmol), 75 g CaBr₂ (375 mmol), 25 ml Wasser und 1 g Tetrabutylammoniumbromid (1,5 Mol %) werden 5 Stunden am Rückfluß gekocht. Man gießt in 500 ml Wasser, trennt die organische Phase ab, wäscht die organische Phase halogenidfrei und trocknet mit wenig CaCl₂.
- Ausbeute:: 94 g
- GC:: 1 % Perfluoroctylbromid
96 % Perfluoroctyliodid

### Vergleichsbeispiel 3

Analog Vergleichsbeispiel 2 erhält man mit 1 g Tetrabutylphosphoniumbromid (1,5 Mol %) als Phasentransferkatalysator:
- Ausbeute:: 95 g
- GC:: 1 % Perfluoroctylbromid
97 % Perfluoroctyliodid

## Patentansprüche

1. Verfahren zur Herstellung von fluorierten Alkylbromiden der Formel I
X-CₙF₂ₙ-Br (I)
worin X = H, F oder (F₃C)₂CF- und n = 1 bis 20 bedeuten, ausgehend von fluorierten Alkyliodiden der Formel III
X-CₙF₂ₙ-I (III)
worin X und n wie oben definiert sind; dadurch gekennzeichnet, daß ein fluoriertes Alkyliodid der Formel III mit einem in der Bromidform vorliegenden Phasentransferkatalysator im Molverhältnis 1 : (0,4 bis 3) umgesetzt wird.

2. Verfahren nach Anspruch 1, dadurch gekennzeichnet, daß n 4 bis 16, besonders bevorzugt 6 bis 12, bedeutet.

3. Verfahren nach Anspruch 1 und/oder 2, dadurch gekennzeichnet, daß in den Formeln I und III die Gruppe -CₙF₂ₙ-die Form -(CF₂)ₙ- besitzt und/oder X die Bedeutung (F₃C)₂CF-, vorzugsweise F, besitzt.

4. Verfahren nach einem oder mehreren der Ansprüche 1 bis 3, dadurch gekennzeichnet, daß als Phasentransferkatalystor in der Bromidform ein quaternäres Ammoniumbromid oder vorzugsweise quaternäres Phosphoniumbromid eingesetzt wird.

5. Verfahren nach einem oder mehreren der Ansprüche 1 bis 4, dadurch gekennzeichnet, daß als Phasentransferkatalysator in der Bromidform Tetrabutylphosphoniumbromid eingesetzt wird.

6. Verfahren nach einem oder mehreren der Ansprüche 1 bis 5, dadurch gekennzeichnet, daß das fluorierte Alkyliodid der Formel III und der Phasentransferkatalysator in der Bromidform im Molverhältnis 1 : (0,5 bis 2), vorzugsweise 1 : (0,8 bis 1,4) und ganz besonders bevorzugt im Molverhältnis von etwa 1 : 1 eingesetzt werden.

7. Verfahren nach einem oder mehreren der Ansprüche 1 bis 6, dadurch gekennzeichnet, daß die Umsetzung bei einer Temperatur von 20°C bis zum Siedepunkt des Reaktionsgemisches, vorzugsweise bei einer Temperatur von 50 bis 140°C, durchgeführt wird.

8. Verfahren nach einem oder mehreren der Ansprüche 1 bis 7, dadurch gekennzeichnet, daß nach beendeter Umsetzung das Reaktionsgemisch mit Wasser vermischt wird und das Gemisch dann in eine wäßrige und in eine organische Phase getrennt wird und daß dann die organische Phase destillativ in ihre Bestandteile zerlegt wird.

9. Verfahren nach einem oder mehreren der Ansprüche 1 bis 8, dadurch gekennzeichnet, daß bei der Aufarbeitung zurückgewonnenes, fluoriertes Alkyliodid der Formel III von neuem als Ausgangsprodukt eingesetzt und/oder bei der Aufarbeitung anfallendes Iodid/Bromid-Gemisch des Phasentransferkatalysators wieder in die Bromidform umgewandelt und von neuem eingesetzt wird.

10. Verfahren nach einem oder mehreren der Ansprüche 1 bis 9, dadurch gekennzeichnet,daß als fluoriertes Alkyliodid der Formel III Perfluoroctyliodid eingesetzt wird.

## Claims

1. Process for the preparation of fluorinated alkyl bromides of the formula I
X-CₙF₂ₙ-Br (I)
in which X is H, F or (F₃C)₂CF- and n is 1 to 20 starting from fluorinated alkyl iodides of the formula III
X-CₙF₂ₙ-I (III)
wherein X and n are defined as above; characterised in that a fluorinated alkyl iodide of the formula III is reacted with a phase transfer catalyst present in the bromide form in a molar ratio of 1:(0.4 to 3).

2. Process according to Claim 1, characterised in that n denotes 4 to 16, particularly preferably 6 to 12.

3. Process according to Claim 1 and/or 2, characterised in that in formulae I and III the group -CₙF₂ₙ- has the form - (CF₂)ₙ - and/or X has the meaning (F₃C)₂CF -, preferably F.

4. Process according to one or more of Claims 1 to 3, characterised in that the phase transfer catalyst used in the bromide form is a quaternary ammonium bromide or preferably quaternary phosphonium bromide.

5. Process according to one or more of Claims 1 to 4, characterised in that the phase transfer catalyst used in the bromide form is tetrabutylphosphonium bromide.

6. Process according to one or more of Claims 1 to 5, characterised in that the fluorinated alkyl iodide of the formular III and the phase transfer catalyst in the bromide form are used in a molar ratio of 1:(0.5 to 2), preferably 1:(0.8 to 1.4) and very particularly preferably in a molar ratio of about 1:1.

7. Process according to one or more of Claims 1 to 6, characterised in that the reaction is carried out at a temperature of 20° C up to the boiling point of the reaction mixture, preferably at a temperature of 50 to 140° C.

8. Process according to one or more of Claims 1 to 7, characterised in that after the reaction is complete the reaction mixture is mixed with water and the mixture is then separated into an aqueous and an organic phase and the organic phase is then separated into its components by distillation.

9. Process according to one or more of Claims 1 to 8, characterised in that fluorinated alkyl iodide of the formula III recovered during work-up is again used as starting material and/or the iodide/bromide mixture of the phase transfer catalyst formed during work-up is reconverted into the bromide form and used again.

10. Process according to one or more of Claims 1 to 9, characterised in that perfluorooctyl iodide is used as fluorinated alkyl iodide of the formula III.

## Revendications

1. Procédé pour la préparation de bromures d'alkyles fluorés de formule I
X-CₙF₂ₙ-Br (I)
où X = H, F ou (F₃C)₂CF- et n=1-20, en partant d'iodures d'alkyle fluorés de formule III
X-CₙF₂ₙ-I (III)
où X et n ont la définition donnée ci-dessus, caractérisés en ce qu'on fait réagir un iodure d'alkyle fluoré de formule III avec un catalyseur de transfert de phases, présent sous forme de bromure, dans un rapport molaire 1 : (0,4 à 3).

2. Procédé selon la revendication 1, caractérisé en ce que n va de 4 à 16, de manière particulièrement préférée de 6 à 12.

3. Procédé selon la revendication 1 et/ou 2, caractérisé en ce que dans les formules I et III, le groupe -CₙF₂ₙ- a la forme -(CF₂)ₙ- et/ou X signifie (F₃C)₂CF-, de préférence F.

4. Procédé selon une ou plusieurs des revendications 1 à 3, caractérisé en ce qu'on utilise en tant que catalyseur de transfert de phases sous la forme de bromure, un bromure d'ammonium quaternaire ou de préférence, un bromure de phosphonium quaternaire.

5. Procédé selon une ou plusieurs des revendications 1 à 4, caractérisé en ce qu'on utilise en tant que catalyseur de transfert de phases sous la forme de bromure, le bromure de tétrabutylphosphonium.

6. Procédé selon une ou plusieurs des revendications 1 à 5, caractérisé en ce qu'on utilise l'iodure d'alkyle fluoré de formule III et le catalyseur de transfert de phases sous la forme de bromure, dans un rapport molaire de 1 : (0,5 à 2), de préférence de 1 : (0,8 à 1,4) et de manière particulièrement préférée dans un rapport molaire d'environ 1 : 1.

7. Procédé selon une ou plusieurs des revendications 1 à 6, caractérisé en ce qu'on met en oeuvre la réaction à une température de 20°C jusqu'au point d'ébulliton du mélange réactionnel, de préférence à une température de 50 à 140°C.

8. Procédé selon une ou plusieurs des revendications 1 à 7, caractérisé en ce que, après avoir terminé la réaction, on mélange le mélange réactionnel avec de l'eau et on sépare le mélange en une phase aqueuse et une phase organique et en ce qu'on fractionne la phase organique en ses composants.

9. Procédé selon une ou plusieurs des revendications 1 à 8, caractérisé en ce que l'iodure d'alkyle fluoré de formule III récupéré au cours du traitement complémentaire est réutilisé en tant que produit de départ et/ou on convertit à nouveau le mélange d'iodure/bromure du catalyseur de transfert de phases formé au cours du traitement complémentaire en la forme bromure et on le réutilise

10. Procédé selon une ou plusieurs des revendications 1 à 9, caractérisé en ce qu'on utilise en tant que iodures d'alkyles fluorés de formule III les iodures de perfluorooctyle.
